(19) Europäisches Patentamt
European Patent Office
Office européen des brevets

(11) EP 2 897 967 B1

(12) EUROPEAN PATENT SPECIFICATION

(45) Date of publication and mention of the grant of the patent:
**21.06.2017 Bulletin 2017/25**

(21) Application number: **13783074.1**

(22) Date of filing: **20.09.2013**

(51) Int Cl.:
***C07F 5/00*** *(2006.01)*
***A61K 49/00*** *(2006.01)*
***G01N 33/58*** *(2006.01)*
***C07D 213/36*** *(2006.01)*
***C09K 11/07*** *(2006.01)*
***C09K 11/06*** *(2006.01)*
***C12N 15/11*** *(2006.01)*
***G01N 33/533*** *(2006.01)*

(86) International application number:
**PCT/FI2013/050912**

(87) International publication number:
**WO 2014/044916 (27.03.2014 Gazette 2014/13)**

(54) **CHELATES, CHELATING AGENTS, CONJUGATES DERIVED THEREOF AND THEIR USE**

CHELATE, CHELATBILDNER, DAVON ABGELEITETE KONJUGATE UND DEREN VERWENDUNG

CHÉLATES, AGENTS CHÉLATANTS, CONJUGUÉS ISSUS DE CEUX-CI ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.09.2012 FI 20120311**

(43) Date of publication of application:
**29.07.2015 Bulletin 2015/31**

(73) Proprietor: **Wang, Qi**
**20610 Turku (FI)**

(72) Inventor: **Wang, Qi**
**20610 Turku (FI)**

(74) Representative: **Seppo Laine Oy**
**Itämerenkatu 3 B**
**00180 Helsinki (FI)**

(56) References cited:
**WO-A1-2010/055207**

- **HAKALA H ET AL: "Development of luminescent Sm(III) chelates containing hexadentate to nonadentate ligands: synthesis, photophysical properties and coupling to biomolecules", JOURNAL OF LUMINESCENCE, ELSEVIER BV NORTH-HOLLAND, NL, vol. 113, no. 1-2, 1 May 2005 (2005-05-01) , pages 17-26, XP027659710, ISSN: 0022-2313 [retrieved on 2005-05-01]**
- **QI WANG ET AL: "Stable and Highly Fluorescent Europium(III) Chelates for Time-Resolved Immunoassays", INORGANIC CHEMISTRY, vol. 52, no. 15, 5 August 2013 (2013-08-05), pages 8461-8466, XP055093775, ISSN: 0020-1669, DOI: 10.1021/ic400384f**
- **"Protecting group", , 30 June 2016 (2016-06-30), Retrieved from the Internet: URL:https://en.wikipedia.org/wiki/Protecting_group [retrieved on 2016-09-29]**
- **F. Eckstein: "Oligonucleotides and Analogues - A practical approach", 1991, IRL Press**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 897 967 B1

## Description

FIELD

This invention relates to stable luminescent lanthanide(III) chelates and biomolecules labeled with these chelates. This invention further relates to the use of the chelates in a method of carrying out a biospecific binding assay.

BACKGROUND

Luminescent lanthanide(III) chelates have several special properties that make them excellent tools in especially homogenous bioaffinity assays. Their large Stokes' shift has a decreasing effect on scattering phenomena. The long fluorescence decay after excitation of these molecules allow time-resolved signal detection, which eliminates completely the background luminescence originating, e.g. from buffer components, plastics, and biomaterials. The very narrow emission lines allow the use of effective filters which diminish the background. Furthermore, since the lanthanide(III) chelates do not suffer from concentration quenching it is possible to have several chelates in close proximity enabling multilabeling. This phenomenom allows also the development of chelates bearing several light absorbing moieties.

However, the use of stable chelates in bioaffinity assays demands optimization of the chelate structure. The optimal luminescent lanthanide chelate must be stable in the presence of additional chelators even at low pH and high temperature. The chelate should have optimal emission profile, high hydrophilicity, small size, good biocompatibility, little effect on biomolecules and good energy transfer properties. In addition, the chelate should be feasible for different energy acceptors, and when possible, the synthesis of the molecule should be simple, cheap and scalable.

Europium(III) chelate of 4-[2-(4-isothiocyanatophenyl)ethynyl]-2,6,-bis{[*N,N*-bis(carboxymethyl)-amino]methyl}pyridine [US 4,920,195] is one of the most commonly used biomolecule labeling reagent, since it fulfills almost all of the above mentioned requirement. The most serious drawback of this chelate as well as other 7-dentate lanthanide(III) chelates is that it exhibits rather low chelate stability limiting its use in applications involving treatments at elevated temperature, low pH and in the presence of additional chelating agents such as EDTA.

The chelate stability can be increased by using cyclic chelating moieties [US 4,920,195; WO2005058877; WO2005021538] but neutralization of the net charge decreases the water solubility of the chelate. The decreased solubility of the cyclic chelates can be enhanced by using hydrophilic groups at the aromatic unit [WO2009030819]. The chelate stability can be enhanced also by increasing the number of chelating carboxylic acids [WO2008020113, US2004166585]. However, all the prior art methods for increasing chelate stability of chelates including pyridine subunit also increase the size of the chelate and/or decrease the chelate solubility.

WO2010055207 discloses chelating agents and chelates including 4-(N-alkylpyrrolo)pyridine subunit, biomolecules labeled with these chelates and chelating agents as well as solid supports conjugated with these chelates, chelating agents and labeled biomolecules.

Hakala et al. discloses development of luminescent Sm(III) chelates containing hexadentate to nonadentate ligands (J. Luminescence, 113, no 1-2, 2005, pp 17-26).

SUMMARY

An object of the present technology is to alleviate and eliminate the problems related to the luminescent lanthanide chelates of prior art.

According to one aspect this technology concerns chelates including a lanthanide(III) ion selected from europium, terbium, samarium and dysprosium and a chelating ligand of formula (I)

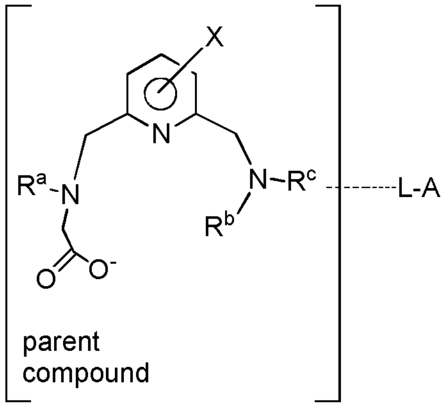

(I)

wherein

X is selected from phenylethynyl, furyl, thienyl, phenyl, and pyrrole groups, and their substituted derivatives, wherein said substituents are selected from alkyl groups and alkoxy groups,

L is a linker formed from one to ten moieties, each moiety being selected from the group consisting of phenylene, alkylene containing 1-12 carbon atoms, ethynydiyl (-C=C-), ethylenediyl (-C=C-), ether (-O-), thioether (-S-), amide (-CO-NH-, -CO-NR'-, -NH-CO- and -NR'-CO-), carbonyl (-CO-), ester (-COO- and -OOC-), disulfide (-SS-), sulfonamide ($-SO_2-NH-$, $-SO_2-NR'-$), sulfone ($-SO_2-$), phosphate ($-O-PO_2-O-$), diaza (-N=N-), and tertiary amine, wherein R' represents an alkyl group containing less than 5 carbon atoms, and L replacing a hydrogen anywhere in the parent compound, or not present,

A is a reactive group selected from isothiocyanate, bromoacetamido, iodoacetamido, maleimido, 4,6-dichloro-1,3,5-triazin-2-ylamino, pyridyldithio, thioester, aminooxy, azide, hydrazide, amino, alkyne, a methacroyl group, carboxylic acid, acid halide, aryl ester, vinyl ester, and hydroxyamine ester,

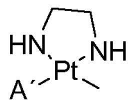

wherein A' is cleaving group selected from Cl, $(CH_3)_2SO$, $H_2O$, and $NO_3$ wherein - is the position of the linker L and

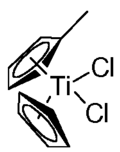

wherein - is the position of linker L,

and A replacing a hydrogen of the X when the linker L is not present, and

$R^a$ is selected from $-CH_2COO-$ and $--(CH_2)_nN(CH_2COO^-)_2$, wherein n is 2 or 3, and

$R^b$ is$-(CH_2)_mN(CH_2COO^-)_2$, wherein m is 2 or 3, and

$R^c$ is selected from $CH_2COO-$, and $--(CH_2)_lN(CH_2COO^-)_2$, wherein l is 2 or 3.

According to another aspect this technology concerns a detectable molecule such as a biomolecule conjugated with a chelate according the present technology wherein the biomolecule is selected from the group consisting of oligopeptide, oligonucleotide, DNA, RNA, modified oligo- or polynucleotide, protein, oligosaccharide, polysaccharide, phospholipide, PNA, LNA, antibody, antigen, steroid, biotin, hapten, drug, receptor bindig ligand, and lectine.

According to another aspect the present technology concerns a method of carrying out a specific bioaffinity assay using a biomolecule conjugate of the present technology with an analyte to be determined.

According to another aspect the present technology concerns a use of a biomolecule conjugate according to the present technology in a specific bioaffinity binding assay utilizing fluorometric or time-resolved fluorometric determination of a specific luminescence.

Further aspects of the present technology have been disclosed in dependent claims.

BRIEF DESCRIPTION OF DRAWINGS

Figure 1 discloses the structures of the chelate of prior art (**1**) and two representative chelates according to the present technology (**6**,**7**).

Figure 2 discloses UV-Vis absorption, excitation ($\lambda_{em}$ = 615 nm) and emission ($\lambda_{exc}$ = 319 nm) spectra of **1b** in 0.01 M TRIS/HCl buffer at pH 7.4.

Figure 3 discloses emission spectra of the **1b**, **6b** and **7b** complexes (from bottom to top) in TRIS/HCl buffer (0.01 M, pH 7.4; $\lambda_{exc}$ = 318 nm).

Figure 4 discloses hCRP immunoassays with biotin-conjugated europium chelates: **1c** (■), **6c** (●), and **7c** (▲). The lower limit of detections for the biotin chelates were 6.5, 1.5 and 1.9 μg/L, respectively.

DETAILED DESCRIPTION

In this invention, it was observed that stability of a lanthanide chelate including a pyridine subunit can be enhanced without need to increase significantly the size of the chelating ligand. According to one embodiment, the present technology concerns chelates including a lanthanide(III) ion selected from europium, terbium, samarium and dysprosium and a chelating ligand of formula (I)

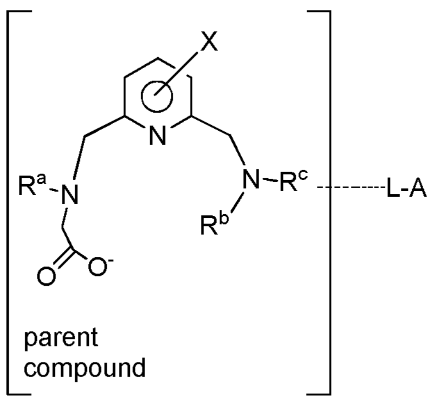

(I)

wherein

X is selected from phenylethynyl, furyl, thienyl, phenyl, and pyrrole groups, and their substituted derivatives, wherein said substituents are selected from alkyl groups and alkoxy groups.

L is a linker formed from one to ten moieties, each moiety being selected from the group consisting of phenylene, alkylene containing 1-12 carbon atoms, ethynydiyl (-C=C-), ethylenediyl (-C=C-), ether (-O-), thioether (-S-), amide (-CO-NH-, -CO-NR'-, -NH-CO- and -NR'-CO-), carbonyl (-CO-), ester (-COO- and -OOC-), disulfide (-SS-), sulfonamide ($-SO_2$-NH-, $-SO_2$-NR'-), sulfone ($-SO_2$-), phosphate (-O-$PO_2$-O-), diaza (-N=N-), and tertiary amine, wherein R' represents an alkyl group containing less than 5 carbon atoms, and L replacing a hydrogen anywhere in the parent compound or not present,

A is a reactive group selected from isothiocyanate, bromoacetamido, iodoacetamido, maleimido, 4,6-dichloro-1,3,5-triazin-2-ylamino, pyridyldithio, thioester, aminooxy, azide, hydrazide, amino, alkyne, a methacroyl group, carboxylic acid, acid halide, and an active ester,

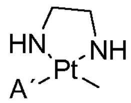

wherein A’ is cleaving group selected from Cl, $(CH_3)_2SO$, $H_2O$, and $NO_3$ wherein - is the position of the linker L and

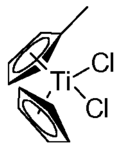

wherein - is the position of linker L,

and A replacing a hydrogen of the X when the linker L is not present, and

$R^a$ is selected from $-CH_2COO^-$ and $-(CH_2)_nN(CH_2COO^-)_2$, wherein n is 2 or 3, and

$R^b$ is $-(CH_2)mN(CH_2COO^-)_2$, wherein m is 2 or 3, and

$R^c$ is selected from $CH_2COO$-, and $-(CH_2)_lN(CH_2COO^-)_2$, wherein l is 2 or 3.

According to a preferable embodiment $R^a$ is selected from $-CH_2COO^-$ and $-CH_2CH_2N(CH_2COO^-)_2$, $R^b$ is $-(CH_2)_2N(CH_2COO^-)_2$, and $R^c$ is $CH_2COO^-$. According to this embodiment the chelates of the present technology have the following structures

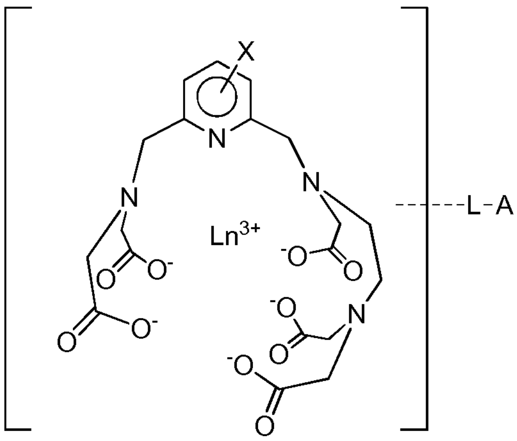

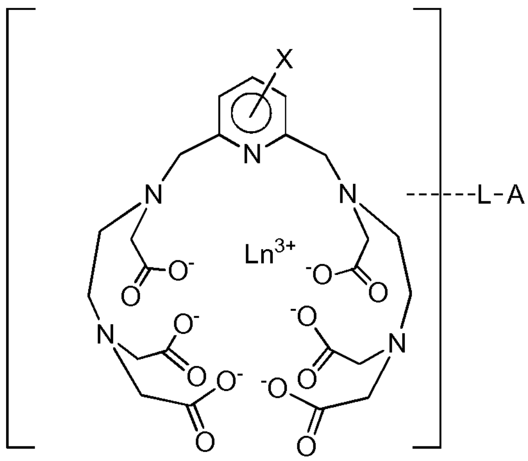

Wherein X, L and A are as defined above, and Ln is selected from Eu, Tb, Sm, and Dy.

As defined herein, the "parent compound" is the part of the chelating ligand of formula (I) that is between the square brackets. Accordingly, the parent compound is understood as

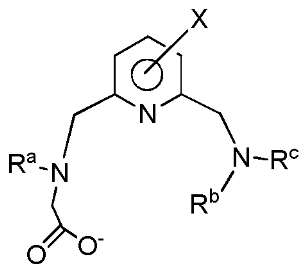

Groups X suitable for the present technology are phenylethynyl, furyl, thienyl, phenyl, and pyrrole groups, and their substituted derivatives. Exemplary substituents are alkyl groups and alkoxy groups. Methods to synthesize the pyridines with group X are known in the art. Exemplary methods are disclosed in Bioconjugate Chemistry, 2009, vol 20, page 405, Scheme 1.

As defined herein, "alkyl group" is linear or branched, like methyl, ethyl, *n*-propyl, *i*-propyl, *n*-butyl, *t*-butyl and *sec*-butyl group. The alkyl group can be tethered also to other groups like hydroxyl, carboxylic acid, carbohydrate and sulfonic acid groups.

As defined herein "alkoxy group" can be linear or branched, like methoxy, ethoxy, *n*-propoxy, *i*-propoxy, *n*-butoxyl, *t*-butoxyl and *sec*-butoxy group. The alkoxy group can be tethered also to other groups like hydroxyl, carbohydrate, carboxylic acid and sulfonic acid groups.

Exemplary alkoxy and alkyl substituted aromatic units are

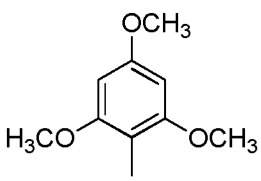 , 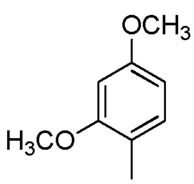 , 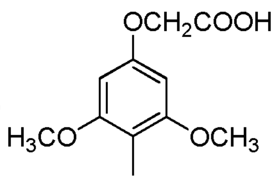 and 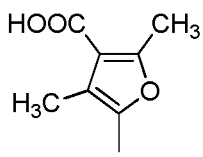

wherein - is the position of wherein the aromatic unit is connected to the pyridine unit and wherein -$CH_2COOH$ and -COOH groups are exemplary reactive groups A.

Group X is connected to 3- or preferably to 4-position of the pyridine unit.

The chelate must bear a reactive group A in order to enable covalent binding to a detectable molecule such as to a biomolecule.

According to an embodiment, the reactive group A is selected from the group consisting isothiocyanate, bromoacetamido, iodoacetamido, maleimido, 4,6-dichloro-1,3,5-triazin-2-ylamino, pyridyldithio, thioester, aminooxy, azide, hydrazide, amino, alkyne, a polymerizable group such as methacroyl group, and a carboxylic acid or carboxylic acid halide or an active ester thereof. As defined herein "active ester" is an aryl ester, vinyl ester, or hydroxyamine ester. Exemplary active esters are nitrophenyl ester, pentafluorophenyl ester and *N*-hydroxysuccinimidyl ester.

It has been proposed [U.S. 5,985,566] that oligonucleotides, DNA, RNA, oligopeptides, proteins and lipids can be transformed statistically by using label molecules tethered to platinum derivatives. In nucleic acids these molecules react predominantly at N7 of guanine residues. When the chelates of the present technology are used for these labelling purposes an exemplary reactive group A is

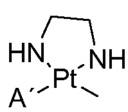

wherein A' is cleaving group selected from Cl, $(CH_3)_2SO$, $H_2O$, and $NO_3$ wherein - is the position of the linker L.

When the chelate of the present technology is used for bioconjugation of phosphodiester linkages an exemplary reactive group A is

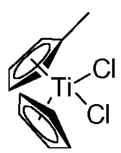

wherein - is the position of linker L.

In case the chelate should be attached to a microparticle or nanoparticle during the manufacturing process of said particles, the reactive group A is a polymerizable group, such as methacroyl group.

In the case the chelate is to be attached to solid supports including nanomaterials, biomolecules, and various organic molecules using copper(I) catalyzed Huisgen-Sharpless dipolar [2+3] cycloaddition reaction, the reactive group A has to be either azide or terminal alkyne.

The reactive group A can be attached to the parent compound either directly or via a linker L. When the reactive group is attached to the parent compound directly, the preferable position is the group X. Exemplary positions are 3- and 4-positions of phenylethynyl unit. According to a preferable embodiment the reactive group A is attached to 4-position of phenylethynyl group and it is selected from amino, isothiocyanate, iodoacetamido and 4,6-dichloro-1,3,5-triazin-2-ylamino groups. According to exemplary embodiments wherein the reactive groups are linked directly to the X are

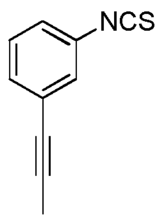

and

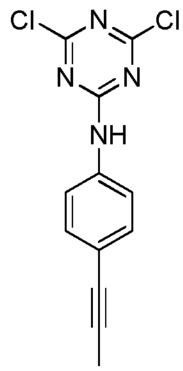

wherein - is the position of pyridine unit and X is phenylethynyl group.

According to one embodiment the reactive group is attached to the parent compound via a liker L. The linker is formed from one to ten moieties, each moiety being selected from the group consisting of phenylene, alkylene containing 1-12 carbon atoms, ethynydiyl (-C=C-), ethylenediyl (-C=C-), ether (-O-), thioether (-S-), amide (-CO-NH-, -CO-NR'-, -NH-CO- and -NR'-CO-), carbonyl (-CO-), ester (-COO- and -OOC-), disulfide (-SS-), sulfonamide ($-SO_2-NH-$, $-SO_2-NR'-$), sulfone ($-SO_2-$), phosphate ($-O-PO_2-O-$), diaza (-N=N-), and tertiary amine, wherein R' represents an alkyl group containing less than 5 carbon atoms and replacing a hydrogen anywhere in the parent compound. Exemplary positions are the pyridine unit, group X and the $CH_2$ units of the chelating part. The preferable position of the linker is the group X. The linker is preferable in applications wherein a space is needed between the chelate and the detectable molecule. An exemplary linker L reactive group A combination is

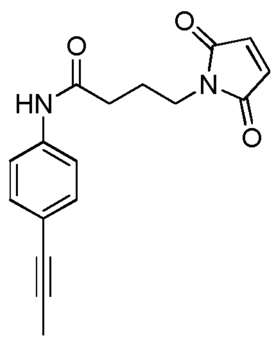

wherein - is the position of the pyridine unit.

Although organic chelators and their substituents have a significant effect on the photophysical properties of lanthanide(III) chelates, no general rules for the estimation of these effects are available. It has been proposed [US 4,761,481] that electron releasing substituents in the aromatic moiety of phenyl and naphthyl substituted 2,6-[N,N-di(carboxyalkyl)aminoalkyl]pyridines have advantageous effects on the photophysical properties on their chelates with lanthanide ions. However, no experimental evidence was given. Later it has been shown that this is the case with various terbium(III) and dysprosium(III) chelates [U.S. patent application Ser. No. 11/004,061] but the corresponding europium(III) chelates are practically non-luminescent [Hemmilä et al., J. Biochem. Biophys. Methods, 1993, 26, 283]. Accordingly, it is clear for a person skilled in art that the choose of the lanthanide ion depends on the nature of the chromophore.

Exemplary chelates according to the present technology has the chelating ligand of formula (III)

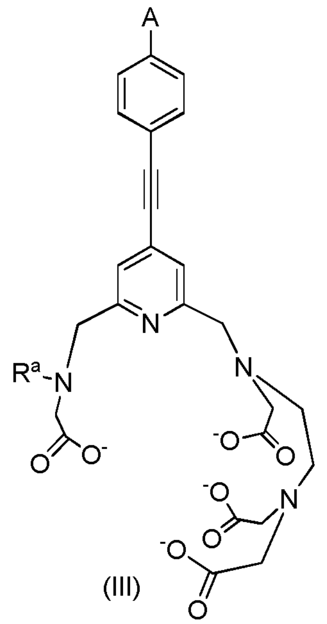

(III)

wherein the reactive group A is selected from amino, iodoacetamido, isothiocyanato and 4,6-dichloro-1,3,5-triazin-2-ylamino, and the lanthanide is selected from europium and samarium, preferably europium, and $R^a$ is selected from $-CH_2COO^-$ and $-CH_2CH_2N(CH_2COO^-)_2$. According to an embodiment, $R^a$ is $CH_2COO^-$.

It is apparent that although the chelating carboxylic acid groups of the chelating ligands of formulas of this disclosure, such as formula (I) and formula (III), are drawn as deprotonated (*i.e.* $-COO^-$), the formulas are only illustrative and the scope of the invention covers chelating ligands wherein one or more of the carboxylic acid groups are protonated (i.e. -COOH).

Exemplary chelates according to the present technology have the chelating ligand of formula (IV) and wherein the reactive group A is selected from amino, iodoacetamido, isothiocyanato and 4,6-dichloro-1,3,5-triazin-2-ylamino, and carboxyl group, and the lanthanide is selected from terbium and dysprosium. According to an embodiment the lanthanide is terbium.

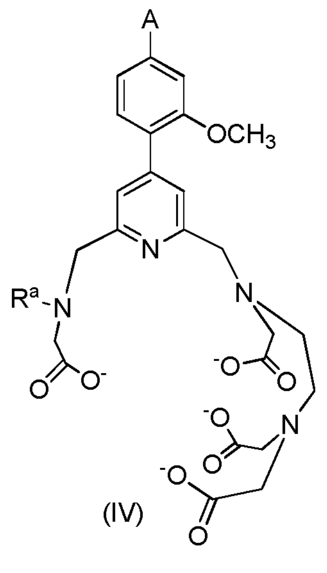

(IV)

Exemplary chelates according to the present technology are

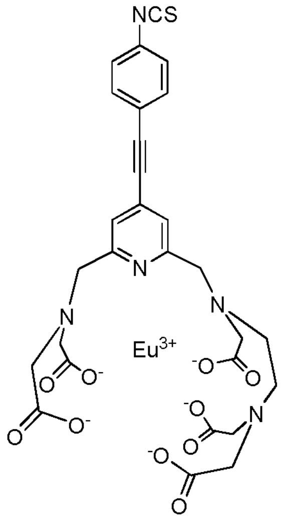

and

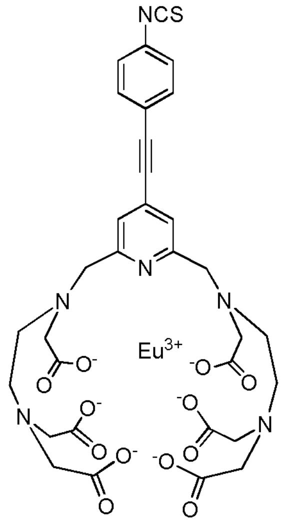

According to another embodiment the present technology concerns a detectable molecule such as a biomolecule conjugated with a chelate according to the present technology. The biomolecule is selected from the group consisting of oligopeptide, oligonucleotide, DNA, RNA, modified oligo- or polynucleotide, protein, oligosaccharide, polysaccharide, phospholipide, PNA, LNA, antibody, antigen, steroid, biotin, hapten, drug, receptor bindig ligand, and lectine. The biomolecule can be labelled with the chelate of the present technology using methods known in the art. The position of labelling and the number of chelates conjugated can be chosen by reaction conditions employed ad by choosing the reactive group A according to the demands of the application and the detectable molecule to be labelled.

The biomolecule conjugated with a chelate according to this invention is an oligopeptide, oligonucleotide, DNA, RNA, modified oligo- or polynucleotide, such as phosphoromonothioate, phosphorodithioate, phosphoroamidate and/or sugar- or base modified oligo- or polynucleotide, protein, oligosaccaride, polysaccaride, phospholipide, PNA, LNA, antibody, steroid, hapten, drug, receptor binding ligand and lectine.

According to another embodiment the present technology concerns a method of carrying out a specific bioaffinity assay using a biomolecule conjugated with a chelate of the present technology with an analyte to be determined.

According to another embodiment the present technology concerns use of a biomolecules conjugated with the chelates of the present technology in a specific bioffinity binding assay utilizing fluorometric or time-resolved fluorometric determination of a specific luminescence. The specific bioffinity assay is preferably selected from selected from a heterogenous immunoassay, a homogenous immunoassay, a DNA hydridization assay, a receptor binding assay, an immunological assay and an immunohistochemical assay.

The essential difference in the structure of the chelates prior art including a single pyridine subunit and the chelates of the present technology can be seen in Fig.1. Although there is no desire to be related to any theory, it is thought that the presence of additional carboxylic acid chelating groups enhance the stability and quantum yield compared to the chelates of the art comprising similar chromophore.

The invention will be illuminated by the following non-restrictive examples.

EXAMPLES

The synthetic routes employed in the experimental part are depicted in Scheme 1. Formulas of compounds **6a-c** and **7a-c** are shown in Figure 1.

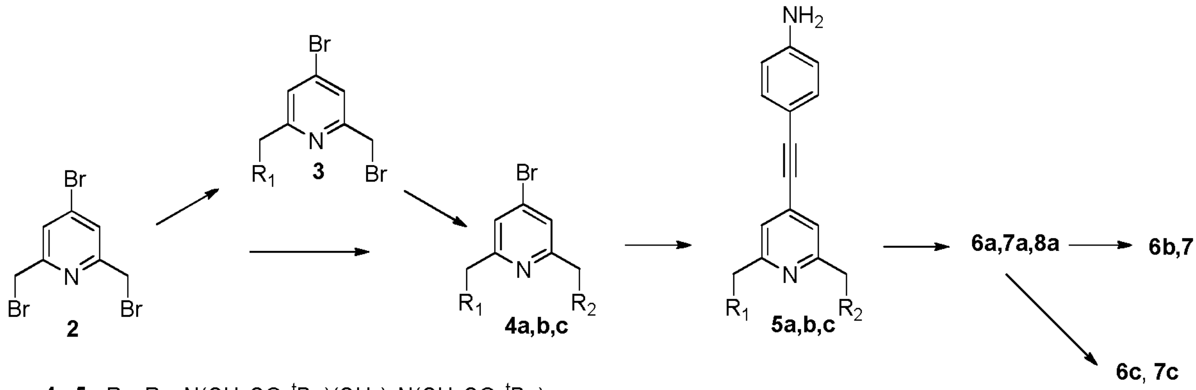


**4a,5a** $R_1=R_2=N(CH_2CO_2{}^tBu)(CH_2)_2N(CH_2CO_2{}^tBu)_2$
**4b,5b** $R_1=N(CH_2CO_2{}^tBu)_2$
$R_2=N(CH_2CO_2{}^tBu)(CH_2)_2N(CH_2CO_2{}^tBu)_2$
**4c,5c** $R_1 =N(CH_2CO_2{}^tBu)_2$
$R_2 = N[(CH_2CH_2)N(CH_2CO_2{}^tBu)_2]_2$

**Scheme 1**

Experimental Procedures

All reagents and solvents used were of reagent grade. The $^1H$ and $^{13}C$ NMR spectra were recorded on a Bruker Avance 600 MHz NMR spectrometer operating at 600.1337 MHz and 150.9179 MHz for $^1H$ and $^{13}C$, respectively. HR Mass spectra were recorded on a Bruker micrOTOF-Q mass spectrometer. UV-visible absorption spectra were recorded on a Specord 205 (Analytik Jena) spectrometer. Steady state emission and excitation spectra were recorded on a Horiba Jobin Yvon Fluorolog 3 spectrometer working with a continuous 450W Xe lamp. Detection was performed with a Hamamatsu R928 photomultiplier. All spectra were corrected for the instrumental functions. When necessary, a 399 nm cut-off filter was used to eliminate the second order artifacts. Phosphorescence lifetimes were measured on the same instrument working in the phosphorescence mode, with 50 μs delay time and a 100 ms integration window.

Emission decay profiles were fitted to mono-exponential and bi-exponential function using the FAST program from Edinburgh Instrument or with the Datastation software from Jobin Yvon. Hydrations numbers, *q*, were obtained using equation (1), were $\tau_{H2O}$ and $\tau_{D2O}$ respectively refer to the measured luminescence decay lifetimes (in ms) in water and deuterated water, using $A_{Eu}$= 1.2 and $a_{Eu}$= 0.25 for $Eu^{III}$:

$$q = A_{Ln}\left(1/\tau_{H_2O} - 1/\tau_{D_2O} - a_{Ln}\right) \qquad (1)$$

Luminescence quantum yields were measured according to conventional procedures, with diluted solutions (optical density <0.05), using $[Ru(bipy)_3]Cl_2$ in nondegassed water (Φ= 4.0%) as reference. The estimated relative error is ± 15%.

Example 1.

Synthesis of Tetra-*tert*-butyl 2,2',2",2'"-{{{[(4-bromopyridine-2,6-diyl)bis(methylene)]bis([2-(*tert*-butoxy)-2-oxoethyl]azanediyl}}bis(ethane-2,1-diyl)}bis(azanetriyl)}tetraacetate (**4a**).

A mixture of 4-bromo-2,6-bis(bromomethyl)pyridine (**2**; 344 mg, 1.0 mmol), *tert*-butyl{[bis(*tert*-butoxycarbonyl methyl)aminoethyl]amino}tris(acetate) (843 mg, 2.10 mmol) and dry potassium carbonate (1.38 g, 10 mmol) in dry acetonitrile (50 mL) was stirred overnight at 55°C. The solid was removed by filtration. The solvent was evaporated *in vacuo* and the product was purified on a silica gel column using 1% (v/v) triethylamine in dichloromethane as the eluent. Yield was 0.56 g (57%). $^1H$ NMR ($CDCl_3$): δ 7.59 (s, 2H), 3.79 (br, 4H), 3.39 (s, 8H), 3.33 (br, 4H), 2.84 (br, 8H), 1.40 (s, 18H), 1.38 (s, 36H). $^{13}C$ NMR ($CDCl_3$): δ 170.48, 160.79, 134.32, 124.09, 80.88, 59.79, 56.05, 53.40, 52.63, 52.06, 28.12, 28.10. HR-MS for $C_{47}H_{81}BrN_5O_{12}{}^+$: required 986.5060 and 988.5040, found 986.4993 and 988.4985.

Example 2

Synthesis of di-*tert*-butyl 2,2'-{{{6-{{{2-(bis[2-(*tert*-butoxy)-2-oxoethyl]amino}ethyl}[2-(*tert*-butoxy)-2-oxoethyl]amino}methyl}-4-bromopyridin-2-yl}methyl}azanediyl}diacetate (**4b**).

Di-*tert*-butyl 2,2'-{[4-bromo-6-(bromomethyl)pyridin-2-yl]methylenenitrilo}bis(acetate) (**3**) (385 mg, 0.76 mmol), *tert*-butyl {[bis(*tert*-butoxycarbonylmethyl)aminoethyl]amino}acetate (319 mg, 0.80 mmol) and potassium carbonate (dry) (524 mg, 3.80 mmol) was stirred overnight at 55°C. The solid was removed by filtration. The solvent was evaporated *in vacuo* and the product was purified on a silica gel column using 20% (v/v) of ethyl acetate in petroleum ether as the eluent. Yield was 562 mg (89%). $^1H$ NMR ($CDCl_3$): $\delta$ 7.69 (s, 1H), 7.59 (s, 1H), 3.94 (s, 4H), 3.41 and 3.40 (2s, 10H), 2.86 (br s, 4H), 1.41 (s, 27H), 1.38 (s, 18H). $^{13}C$ NMR ($CDCl_3$): $\delta$ 170.45, 170.29, 160.60, 134.49, 124.39, 124.26, 81.10, 81.00, 59.65, 59.42, 56.01, 55.81, 52.64, 51.88, 28.13, 28.11. HR-MS for $C_{39}H_{66}BrN_4O_{10}^+$: required 829.3957 and 831.3937, found 829.4050 and 831.4038.

Example 3.

Synthesis of tetra-*tert*-butyl 2,2',2'',2'''-(((((4-((4-aminophenyl)ethynyl)pyridine-2,6-diyl) bis(methylene))bis((2-(tert-butoxy)-2-oxoethyl)azanediyl))bis(ethane-2,1-diyl)) bis(azanetriyl))-tetraacetate (**5a**)

Compound **4a** (364 mg, 0.37 mmol) and 4-ethynylaniline (52 mg, 0.44 mmol) in the mixture of THF (10 mL) and DIPEA (10 mL) was deaerated with nitrogen for 5 min. $Pd(PPh_3)_2Cl_2$ (10.4 mg, 0.015 mmol) and CuI (2.9 mg, 0.015 mmol) were added as the catalysts, and the reaction mixture was stirred overnight under nitrogen at 60°C. The solvents were removed *in vacuo* and the product was purified on a silica gel column using a mixture of 20-30% (v/v) of ethyl acetate in petroleum ether containing 1% (v/v) triethylamine as the eluent. Yield was 290 mg (76%). $^1H$ NMR ($CDCl_3$, 50°C): $\delta$ 7.44 (s, 2H), 7.30 (d, *J*=8.45 Hz, 2H), 6.61 (d, J=8.50 Hz, 2H), 3.96 (br s, 4H), 3.43 (s, 8H), 3.40 (br s, 4H), 2.90 (b, 8H), 1.46 (s, 18H), 1.42 (s, 36H). $^{13}C$ NMR ($CDCl_3$, 50°C): $\delta$ 170.55, 158.78, 147.56, 133.32, 132.57, 122.66, 114.54, 111.47, 85.87, 80.83, 80.75, 60.07, 56.24, 52.88, 52.47, 28.17, 28.13. HR-MS for $C_{55}H_{87}N_6O_{12}^+$: required 1023.6376, found 1023.6308.

Example 4.

Synthesis of di-t*ert*-butyl 2,2'-(((4-((4-aminophenyl)ethynyl)-6-(((2-(bis(2-(*tert*-butoxy)-2-oxoethyl)amino)-ethyl)(2-(*tert*-butoxy)-2-oxoethyl)amino)methyl)pyridin-2-yl)methyl)azanediyl)diacetate (**5b**)

The synthesis was performed as above for compound **5a** but using **4b** (562 mg, 0.68 mmol) as the starting material. Yield was 421 mg (72%). $^1H$ NMR ($CDCl_3$): $\delta$ 7.45 (s, 1H), 7.35 (s, 1H), 7.18 (d, J=8.46 Hz, 2H), 6.51 (d, J=8.52 Hz, 2H), 4.17 (br s, 2H), 3.91 (s, 2H), 3.81 (s, 2H), 3.38 (s, 4H), 3.35 (s, 4H), 3.27 (s, 2H), 2.77 (t, d, J=6.54, 25.21 Hz, 4H), 1.37 (s, 27H), 1.33 (s, 18H). $^{13}C$ NMR ($CDCl_3$): $\delta$ 170.68, 170.55, 170.38, 159.06, 158.64, 147.93, 133.18, 133.15, 122.47, 122.38, 114.38, 110.70, 94.80, 85.66, 80.89, 80.72, 80.70, 60.21, 60.08, 59.63, 56.14, 56.09, 55.71, 52.55, 52.16, 28.09, 28.07, 28.04. HR-MS for $C_{47}H_{72}N_5O_{10}^+$: required 866.5274, found 866.5294.

Example 5.

Preparation of the complex **6a**.

Compound **5b** (220 mg, 0.215 mmol) was dissolved in TFA (2 mL) and the mixture was stirred in a water-bath at 25°C for 2 hours. All volatiles were removed *in vacuo.* The residue was dissolved in water (2 mL), a $EuCl_3$ solution (0.236 mmol in 0.5 mL water) was added and stirred for 10 min. The pH was adjusted to 7.0 with triethylamine and the mixture was stirred for another 10 minutes. The pH was adjusted to 9.0 with sat. $Na_2CO_3$. The precipitate formed was removed by centrifugation. The pH of the solution was adjusted to 7.0 with acetic acid. Acetone (45 mL) was added and the mixture was shaken for 1 min. The precipitate was collected by centrifugation, washed with acetone (50 mL) and dried with airflow. HR-MS for $C_{27}H_{27}EuN_5O_{10}^-$: required 732.0961 and 734.0975, found 732.1024 and 734.1032. The precipitate was dissolved in water (1 mL). Chloroform (1 mL) and thiophosgene (0.33 mL, 4.3 mmol) were added and the mixture was stirred vigorously for 5 minutes. The pH was monitored and kept at 7.0 with 15% of $NaHCO_3$ solution. Chloroform was removed followed by addition of acetone (50 mL) with stirring. The precipitate formed was isolated by centrifugation, washed with acetone (50 mL) and dried. HR-MS for $C_{28}H_{25}EuN_5O_{10}S^-$: required 774.0526 and 776.0539, found 774.0649 and 776.0694.

Example 6.

Preparation of complex **7a**

The title compound was prepared as described above for compound **6a** but using compound **5a** (234 mg, 0.27 mmol) as the starting material. HR-MS for the amino form $C_{31}H_{33}EuN_6O_{12}^{2-}$: required 416.0683 and 417.0690, found 416.0721 and 417.0740. HR-MS for the isothiocyanato form $C_{32}H_{31}EuN_6O_{12}S^{2-}$: required 437.0465 and 438.0472, found 437.0521 and 438.0512.

Example 7.

Preparation of glycine-complexes **1b**, **6b** and **7b**.

The isothiocyanates (**1a**, **6a**, **7a**; 20 mg each) were allowed to react with glycine (300 mg) at pH ca 7. The product was purified by HPLC (column: Supelco Ascentis RP-Amide, 21.2 mm · 25cm. Particle 5μm, flow rate 8.0 mL/min; eluent 20mM TEAA buffer in 2-25% acetonitrile, v/v). The fractions were collected and concentrated. The salts were removed on HPLC by using the above mentioned system by omitting the buffer component from the eluent.

HR-MS for **1b** $C_{26}H_{22}EuN_5O_{10}S^{2-}$: required 373.5148 and 374.5155, found 373.5163 and 374.5173.
HR-MS for **6b** $C_3oH_{29}EuN_60_{12}S^{2}$-: required 424.0387 and 425.0393, found 424.0398 and 425.0380.
HR-MS for **7b** $C_{34}H_{36}EuN_7O_{14}S^{2-}$: required 474.5625 and 475.5632, found 474.5676 and 475.5690.

Example 8.

Preparation of Biotin-complexes of **1c**, **6c** and **7c**.

The isothiocyanates (**1a**, **6a**, **7a**; 1 mg each) were allowed to react with N-Biotinyl-3-aminopropylamine (TFA salt, 5 mg each) at pH ca 8.0. The product was purified by HPLC (column: Supelco Ascentis RP-Amide, 4.6 mm x 15 cm, particle 5μm, flow rate 1.0 mL/min; eluent 20 mM TEAA buffer at pH 7.0 with 2-60% methanol, v/v). The fractions were collected and dried in vacuum.

HR-MS for **1c** $C_{37}H_{41}EuN_8O_{10}S_2^{2-}$: required 486.0798 and 487.0805, found 486.0764 and 487.0780.
HR-MS for **6c** $C_{41}H_{48}EuN_9O_{12}S_2^{2-}$: required 536.6037 and 537.6044, found 536.6014 and 537.6023.
HR-MS for **7c** $C_{45}H_{55}EuN_{10}O_{14}S_2^{2-}$: required 587.1275 and 588.1282, found 587.1251 and 588.1260.

Example 9

Preparation of the complex **8a**

The isothiocyate chelate **8a** is synthesized as described above for **7a** *i.e.* starting from **3**, but using tetra-*tert*-butyl 2,2',2",2"-(azanediylbis(ethane-2,1-diyl))bis(azanetriyl))tetraacetate synthesized as disclosed in Bioconjugate Chem. 2008, 19, 1505-1509.

Example 10.

Conditional stability constants determination.

In a typical experiment batches of 2 mL of *ca* $2\times10^{-5}$ M solutions of the europium complexes in TRIS buffer at pH = 7.4 were mixed with various quantities of a stock solution of $5\times10^{-2}$ M EDTA (or DOTA) in the same buffer, so that the ratio of added EDTA per Eu complex varies from 0 to 450000. For each solution, the emission spectrum was measured and the decrease in intensity was fitted to equation (2) using the non-linear regression analysis of the SPECFIT software

$$[EuL] + EDTA \leftrightarrow [Eu(EDTA)] + L \qquad (2)$$

with

$$K = \frac{[Eu(EDTA)]\times[L]}{[EDTA]\times[EuL]} = \frac{K_{condEDTA}}{K_{condL}}$$

The conditional stability constant could be determined using values of $K_{condEDTA}$ calculated from literature data.

Example 11

hCRP immunoassay.

Human C-reactive protein (CRP) was obtained from (Orion Diagnostica, Espoo, Finland) and monoclonal anti-CRP antibody 6404 was from Medix Biochemica (Kauniainen, Finland). Nunc C12 low fluor maxi wells (Thermo Scientific, Roskilde, Denmark) were coated with 150 ng of 6404 antibody in 40 μl 50 mM phosphate buffer pH 7.4 for 16 h at 4 °C. The wells were washed twice using wash buffer from Kaivogen Oy (Turku, Finland). Final blocking of the well surface was carried out with 200 μl 0.1 % BSA in the phophate buffer for 2 hours at 25 °C. The same 6404 antibody was conjugated with *d*-biotin NHS ester (Sigma-Aldrich, St. Louis, USA) using 20-fold excess in 50 mM phosphate buffer pH 7.8. After conjugation the antibody was purified with NAP-5 column (GE Healthcare, Uppsala, Sweden) using TBS-buffer (50 mM Tris-Cl pH 7.5, 150 mM NaCl). Three replicates of 0 - 10 mg/L hCRP were incubated for 60 min in 50 μl of TBS-buffer in prewashed 6404 coated wells. The wells were washed once, and 50 ng of biotinylated 6404 antibody was added to the wells in 50 μl of TBS and incubated for 60 min. The wells were washed once and 50 ng streptavidin (BioSpa, Milan, Italy) in 50 μl of TBS was added, incubated for 10 minutes and washed once. Thereafter, 50 μl of 40 nM biotinylated europium chelates were added and incubated for 10 minutes. The wells were washed twice and measured with a Victor$^2$ 1420 multilabel counter (PerkinElmer, Wallac OY, Turku, Finland) in time-resolved luminescence mode using an excitation wavelength of 340 nm and an emission wavelength of 615 nm, a 400 μs delay, and 400 μs integration times.

Spectroscopic properties of the chelates. The spectroscopic properties of the complexes were measured in 0.01 M TRIS/HCl buffer at pH 7.4 on the glycine functionalized complexes (**1b**, **6b**, **7b**), and the most important parameters are gathered in Table 1.

**Table 1.** Spectroscopic data for the Eu complexes in 0.01M TRIS/HCl buffer at pH 7.4

| Compound | $\lambda_{max}$/ nm | $\varepsilon$/M$^{-1}$.cm$^{-1}$ | $\tau_{H2O}$/ms | $\tau_{D2O}$/ms | $q$ | $\phi_{Ov}$ | $\eta_{eff}$ | $\Phi_{Eu}$ |
|---|---|---|---|---|---|---|---|---|
| **1b** | 319 | 16350 | 0.39 | 2.10 | 2.2 | 0.06 | 0.54 | 0.11 |
| **6b** | 317 | 24350 | 1.15 | 1.96 | 0.1 | 0.14 | 0.45 | 0.31 |
| **7b** | 318 | 20900 | 1.07 | 1.69 | 0.1 | 0.12 | 0.41 | 0.29 |

The UV-Vis absorption spectra of the three Eu complexes are very similar, displaying a strong absorption band centred at *ca* 318 nm, corresponding to $\pi\rightarrow\pi^*$ transitions on the pyridyl rings (see Figure 2 for **1b**). The presence of the *para*-(thiourea)-toluyl substitution resulted in a strong bathochromic shift of this absorption band, when compared to non substituted pyridines for which the maximum of absorption can be found at 265-267 nm. Upon excitation into the $\pi\rightarrow\pi^*$ absorption bands, all complexes display well resolved emission bands between 575 and 730 nm associated to f-f transitions on the europium atom. These emission bands correspond to the $^5D_0\rightarrow{}^7F_J$ transitions with $J$ = 0 (single band at 575 nm), $J$= 1 (between 578 and 600 nm), $J$ = 2 (strong, 605 to 625 nm), $J$ = 3 (weak around 650 nm) and $J$ = 4 (690 to 715 nm). The corresponding excitation spectra are perfectly superimposable with the absorption spectra, evidencing an efficient ligand to metal energy transfer process that is a good antenna effect. The luminescence decay profiles measured at the maximum of emission were all perfectly fitted with mono-exponential functions, pointing to the presence of single species in solution. The complex obtained from the heptadentate ligand of **1b** displayed the shorter lifetime (0.39 ms), the coordination sphere of the europium being probably unsaturated. This was confirmed by the calculation of the hydration numbers of the complexes according to the method developed by Horrocks using Beeby's coefficients (Table 1). While the heptadentate ligand of **1b** releases the place for two inner sphere water molecules, the replacement of one or two acetate functions by iminodiacetate ones resulted in the fulfilment of the coordination sphere and the removal of solvent molecules from the first coordination sphere.

The Eu centred emission spectra of the complexes are presented in Figure 3. The $^5D_0\rightarrow{}^7F_2$ transition represents the most intense emission band. The main variations in the series are observed on this transition and on the pattern of the $^5D_0\rightarrow{}^7F_4$ transition, this transition becoming more intense (compared those with $J$ = 0 to 3) for **7b**.

Based on the emission spectra of the three complexes (Figure 2), it was possible to determine the europium

centred quantum yield, $\Phi_{Eu}$ from which one can calculate the sensitization efficiency, $\eta_{eff}$, that reflects the capacity of the ligand to transfer the absorb energy to the europium, using equation

$$\Phi_{Ov} = \eta_{eff} \times \Phi_{Eu}$$

Where $\Phi_{Ov}$ is the overall luminescence quantum yield measured by direct method (Table 1).

The calculated values of $\eta_{eff}$ and $\Phi_{Eu}$ evidenced that the sensitization is almost the same for all compounds ranging from 41 to 54 %, as expected for a similar antenna unit. In contrast, the metal centred quantum yield of **1b** is largely affected by the presence of the two inner sphere water molecules, losing two third of the value obtained when the coordination sphere is saturated.

Determination of the conditional stability constants. The determination of the conditional stability constants of the different complexes was addressed by means of competition experiments with EDTA and DOTA. Only in the case of **1b** it was possible to displace the equilibrium in the presence of EDTA. Considering a conditional stability constant of 14.53 Log units for EDTA at this pH, the fitting resulted in a conditional stability constant of 16.7Log units for **1b** at pH = 7.4. For both **6b** and **7b**, even in the presence of large excess of EDTA, it was not possible to displace the equilibrium, and to extract the Eu atom from its coordinating ligand. Even DOTA which is known to form very stable complexes with Ln cations was not able to demetallate the chelates of the present technology even after three days at 80°C.

Conjugation to Bioactive Molecules and hCRP immunoassay. The applicability of the chelates was demonstrated in a sandwich-type hCRP immunoassay. Therefore, the chelates **1b, 6b** and **7b** were conjugated with N-Biotinyl-3-aminopropylamine. The synthesized chelates **6c** and **7c** performed equally well in the hCRP assay. The biotin-chelate **1c** resulted in lower luminescence signal than the **6c** and **7c** which is in accordance with the measured quantum yields and emission lifetimes. The analytical detection limits for **1c**, **6c** and **7c** were 6.5, 1.5 and 1.9 μg/L respectively as calculated from 3 SD above the mean of the zero hCRP concentration and from the equations **1c**: y = 32515x - 34, $R^2$ = 0,96; **6c**: y = 87730x +16, $R^2$ = 0,99; **7c**: y= 90760x - 31, $R^2$ = 0,99.

## Claims

1. A chelate comprising a lanthanide(III) ion selected from europium, terbium, samarium and dysprosium and a chelating ligand of formula (I)

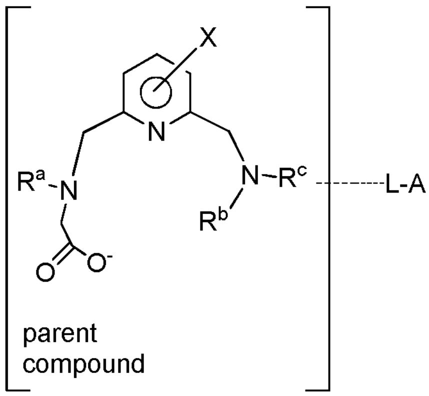

(I)

wherein

X is selected from phenylethynyl, furyl, thienyl, phenyl, and pyrrole, and their substituted derivatives and wherein said substituents are selected from alkyl groups and alkoxy groups,

L is a linker formed from one to ten moieties, each moiety being selected from the group consisting of phenylene, alkylene containing 1-12 carbon atoms, ethynydiyl (-C=C-), ethylenediyl (-C=C-), ether (-O-), thioether (-S-), amide (-CO-NH-, -CO-NR'-, -NH-CO- and -NR'-CO-), carbonyl (-CO-), ester (-COO- and -OOC-), disulfide (-SS-), sulfonamide ($-SO_2-NH-$, $-SO_2-NR'-$), sulfone ($-SO_2-$), phosphate ($-O-PO_2-O-$), diaza (-N=N-), and tertiary amine, wherein R' represents an alkyl group containing less than 5 carbon atoms, and L replacing a hydrogen anywhere in the parent compound, or not present,

A is a reactive group selected from isothiocyanate, bromoacetamido, iodoacetamido, maleimido, 4,6-dichloro-1,3,5-triazin-2-ylamino, pyridyldithio, thioester, aminooxy, azide, hydrazide, amino, alkyne, a methacroyl group, carboxylic acid, acid halide, aryl ester, vinyl ester, and hydroxyamine ester,

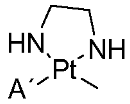

wherein A' is cleaving group selected from Cl, $(CH_3)_2SO$, $H_2O$, and $NO_3$ wherein - is the position of the linker L and

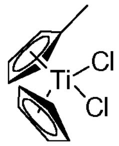

wherein - is the position of linker L,
and A replacing a hydrogen of the group X when the linker L is not present, and
$R^a$ is selected from $-CH_2COO-$ and $-(CH_2)_nN(CH_2COO^-)_2$, wherein n is 2 or 3, and $R^b$ is $-(CH_2)_mN(CH_2COO^-)_2$, wherein m is 2 or 3, and
$R^c$ is selected from $CH_2COO^-$, and $-(CH_2)_lN(CH_2COO^-)_2$, wherein l is 2 or 3.

**2.** The chelate according to claim 1 wherein $R^a$ is selected from $-CH_2COO^-$ and $-CH_2CH_2N(CH_2COO^-)_2$, $R^b$ is $-CH_2COO^-$, and $R^c$ is $-(CH_2)_2N(CH_2COO^-)_2$.

**3.** The chelate according to claim 1 wherein the chelating ligand has the formula (III)

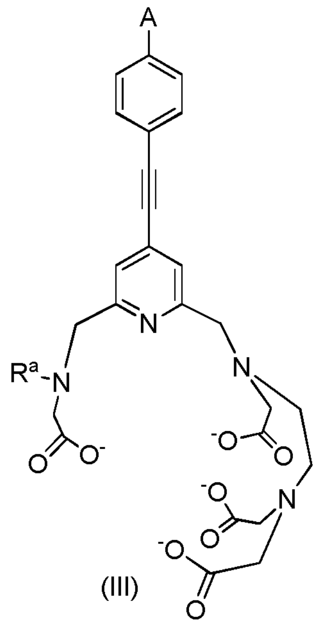

(III)

wherein the reactive group A is selected from amino, iodoacetamido, isothiocyanato and 4,6-dichloro-1,3,5-triazin-2-ylamino, and the lanthanide is selected from europium and samarium.

**4.** The chelate according to claim 2 or 3 wherein the lanthanide is europium.

**5.** The chelate according to claim 1 wherein X is alkoxyphenyl group and the lanthanide is selected from terbium and dysprosium.

**6.** The chelate according to any of claims 1-5 wherein $R^a$ is $-CH_2COO^-$.

7. A biomolecule conjugated with a chelate according to any of claims 1-6 wherein the biomolecule is selected from the group consisting of oligopeptide, oligonucleotide, DNA, RNA, modified oligo- or polynucleotide, protein, oligosaccharide, polysaccharide, phospholipide, PNA, LNA, antibody, antigen, steroid, biotin, hapten, drug, receptor bindig ligand, and lectine.

8. A method of carrying out a specific bioaffinity assay using a biomolecule of claim 7 with an analyte to be determined.

9. Use of a biomolecule according to claim 7 in a specific bioaffinity binding assay utilizing fluorometric or time-resolved fluorometric determination of a specific luminescence.

10. The use according to claim 9 wherein the specific bioffinity assay is selected from a heterogenous immunoassay, a homogenous immunoassay, a DNA hydridization assay, a receptor binding assay, an immunological assay and an immunohistochemical assay.

**Patentansprüche**

1. Chelat umfassend ein Lanthanid(III)-Ion ausgewählt aus Europium, Terbium, Samarium und Dysprosium und einen chelatisierenden Liganden der Formel (I)

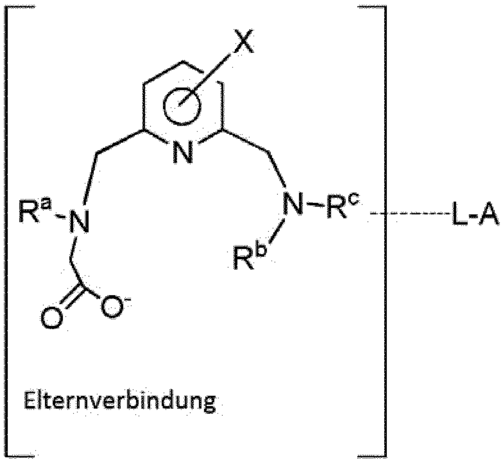

(I)

wobei

X ausgewählt ist aus Phenylethinyl, Furyl, Thienyl, Phenyl, und Pyrrol , und deren substituierten Derivaten und wobei die Substituenten ausgewählt sind aus Alkylgruppen und Alkoxygruppen,
L ein Linker ist, welcher aus ein bis zehn Resten gebildet ist, wobei jeder Rest ausgewählt ist aus der Gruppe bestehend aus Phenylen, Alkylen enthaltend 1 - 12 Kohlenstoffatome, Ethinyldiyl (-C=C-), Ethylendiyl (-C=C-), Ether (-O-), Thioether (-S-), Amid (-CO-NH-, -CO-NR'-, -NH-CO- und -NR'-CO-), Carbonyl (-CO-), Ester (-COO- und -OOC-), Disulfid (-SS-), Sulfonamid ($-SO_2-NH-$, $-SO_2-NR'-$), Sulfon ($-SO_2-$), Phosphat ($-O-PO_2-O-$), Diaza (-N=N-), und tertiärem Amin, wobei R' eine Alkylgruppe enthaltend weniger als 5 Kohlenstoffe darstellt, und L an einer beliebigen Stelle in der Elternverbindung einen Wasserstoff ersetzt, oder nicht vorhanden ist,
A eine reaktive Gruppe ist, welche ausgewählt ist aus Isothiocyanat, Bromacetamido, Iodacetamido, Maleimido, 4,6-Dichlor-1,3,5-triazin-2-ylamino, Pyridyldithio, Thioester, Aminooxy, Azid, Hydrazid, Amino, Alkin, einer Methacroylgruppe, Carbonsäure, Säurehalogenid, Arylester, Vinylester, und Hydroxaminester,

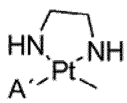

wobei A' eine Abspaltgruppe ist, welche ausgewählt ist aus Cl, $(CH_3)_2SO$, $H_2O$, und $NO_3$, wobei - die Position

des Linkers L ist, und

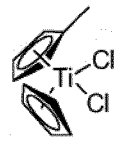

wobei - die Position des Linkers L ist, und A einen Wasserstoff der Gruppe X ersetzt, wenn der Linker L nicht vorhanden ist, und $R^a$ ausgewählt ist aus $-CH_2COO^-$ und $-(CH_2)_nN(CH_2COO^-)_2$, wobei n 2 oder 3 ist, und $R^b$ $-(CH_2)_mN(CH_2COO^-)_2$ ist, wobei m 2 oder 3 ist, und $R^c$ ausgewählt ist aus $-CH_2COO^-$, und $-(CH_2)_lN(CH_2COO^-)_2$, wobei l 2 oder 3 ist.

2. Chelat nach Anspruch 1, wobei $R^a$ ausgewählt ist aus $-CH_2COO^-$ und $-CH_2CH_2N(CH_2COO^-)_2$, $R^b$ $-CH_2COO^-$ ist, und $R^c$ $-(CH_2)_2N(CH_2COO^-)_2$ ist.

3. Chelat nach Anspruch 1, wobei der chelatisierende Ligand die Formel (III) aufweist

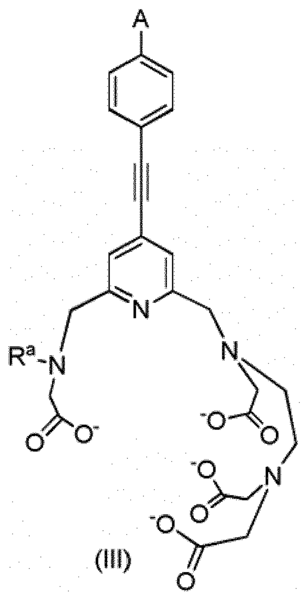

(III)

wobei die reaktive Gruppe A ausgewählt ist aus Amino, Iodacetamid, Isothiocyanato und 4,6-Dichlor-1,3,5-triazin-2-ylamino, und das Lanthanid ausgewählt ist aus Europium und Samarium.

4. Chelat nach Anspruch 2 oder 3, wobei das Lanthanid Europium ist.

5. Chelat nach Anspruch 1, wobei X eine Alkoxyphenylgruppe ist und das Lanthanid ausgewählt ist aus Terbium und Dysprosium.

6. Chelat nach einem der Ansprüche 1 - 5, wobei $R^a$ $-CH_2COO^-$ ist.

7. Biomolekül konjugiert mit eine Chelat nach einem der Ansprüche 1 - 6, wobei das Biomolekül ausgewählt ist aus der Gruppe bestehend aus Oligopeptid, Oligonukleotid, DNA, RNA, modifiziertem Oligo- oder Polynukleotid, Protein, Oligosaccharid, Polysaccharid, Phospholipid, PNA, LNA, Antikörper, Antigen, Steroid, Biotin, Hapten, Wirkstoff, rezeptorbindendem Ligand, und Lektin.

8. Verfahren zum Durchführen eines spezifischen Bioaffinitätsassays unter Verwendung eines Biomoleküls nach Anspruch 7 mit einem zu bestimmenden Analyten.

9. Verwendung eines Biomoleküls nach Anspruch 7 in einem spezifischen Bioaffinitätsbindungsassays unter Verwendung einer fluorometrischen oder zeitaufgelösten fluorometrischen Bestimmung einer spezifischen Lumineszenz.

**10.** Verwendung nach Anspruch 9, wobei das spezifische Bioaffinitätsassay ausgewählt ist aus einem heterogenen Immunassay, einem homogenen Immunassay, einem DNA-Hybridisierungsassay, einem Rezeptorbindungsassay, einem immunologischen Assay und einem immunohistochemischen Assay.

## Revendications

**1.** Chélate comprenant un ion lanthanide (III) choisi parmi l'europium, le terbium, le samarium et le dysprosium et un ligand chélatant de formule (I)

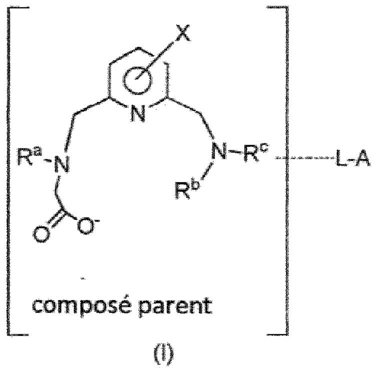

(I)

dans laquelle

X est choisi parmi un groupe phényléthynyle, furyle, thiényle, phényle, et pyrrole, et leurs dérivés substitués et dans laquelle lesdits substituants sont choisis parmi les groupes alkyle et les groupes alcoxyle,
L est un lieur formé d'une à dix fractions, chaque fraction étant choisie dans le groupe constitué par un groupe phénylène, alkylène contenant de 1 à 12 atomes de carbone, éthynydiyle (-C≡C-), éthylènediyle (-C=C-), éther (-O-), thioéther (-S-), amide (-CO-NH-, -CO-NR'-, -NH-CO- et -NR'-CO-), carbonyle (-CO-), ester (-COO- et -OOC-), disulfure (-SS-), sulfonamide ($-SO_2$-NH-, $-SO_2$-NR'-), sulfone ($-SO_2-$), phosphate (-O-$PO_2$-O-), diaza (-N=N-), et amine tertiaire, dans lesquels R' représente un groupe alkyle contenant moins de 5 atomes de carbone, et L remplaçant un hydrogène n'importe où dans le composé parent, ou est absent,
A est un groupe réactif choisi parmi un groupe isothiocyanate, bromoacétamido, iodoacétamido, maléimido, 4,6-dichloro-1,3,5-triazin-2-ylamino, pyridyldithio, thioester, aminooxy, azide, hydrazide, amino, alcyne, un groupe méthacroyle, un acide carboxylique, un halogénure d'acide, un ester d'aryle, un ester vinylique, et un ester d'hydroxyamine,

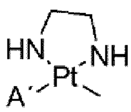

dans lequel A' est un groupe clivant choisi parmi Cl, $(CH_3)_2SO$, $H_2O$, et $NO_3$ dans lequel - est la position du lieur L et

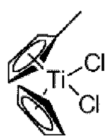

dans lequel - est la position du lieur L,
et A remplaçant un groupe hydrogène du groupe X lorsque le lieur L est absent, et
$R^a$ est choisi parmi les groupes $-CH_2COO^-$ et $-(CH_2)_nN(CH_2COO^-)_2$, dans lequel n est 2 ou 3, et
$R^b$ est un groupe $-(CH_2)_mN(CH_2COO^-)_2$, dans lequel m est 2 ou 3, et

$R^c$ est choisi parmi les groupes $-CH_2COO^-$, et $-(CH_2)_lN(CH_2COO^-)_2$, dans lequel l est 2 ou 3.

**2.** Chélate selon la revendication 1, dans lequel $R^a$ est choisi parmi les groupes $-CH_2COO^-$ et $-CH_2CH_2N(CH_2COO^-)_2$, $R^b$ est un groupe $-CH_2COO^-$, et $R^c$ est un groupe $-(CH_2)_2N(CH_2COO^-)_2$.

**3.** Chélate selon la revendication 1, dans lequel le ligand chélatant a la formule (III)

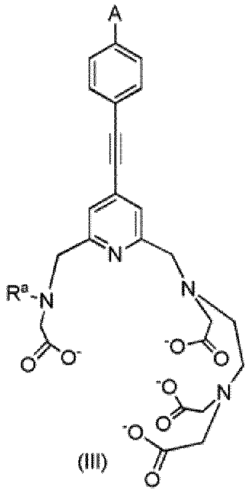

(III)

dans laquelle le groupe réactif A est choisi parmi un groupe amino, iodoacétamido, isothiocyanato et 4,6-dichloro-1,3,5-triazin-2-ylamino, et le lanthanide est choisi parmi l'europium et le samarium.

**4.** Chélate selon la revendication 2 ou 3, dans lequel le lanthanide est l'europium.

**5.** Chélate selon la revendication 1, dans lequel X est un groupe alcoxyphényle et le lanthanide est choisi parmi le terbium et le dysprosium.

**6.** Chélate selon l'une quelconque des revendications 1 à 5, dans lequel $R^a$ est un groupe $-CH_2COO^-$.

**7.** Biomolécule conjuguée avec un chélate selon l'une quelconque des revendications 1 à 6, dans laquelle la biomolécule est choisie dans le groupe constitué par un oligopeptide, un oligonucléotide, un ADN, un ARN, un oligo-ou polynucléotide modifié, une protéine, un oligosaccharide, un polysaccharide, un phospholipide, un PNA, un LNA, un anticorps, un antigène, un stéroïde, la biotine, un haptène, un médicament, un ligand de liaison de récepteur, et une lectine.

**8.** Procédé de mise en oeuvre d'un test de bioaffinité spécifique en utilisant une biomolécule selon la revendication 7 avec un analyte à déterminer.

**9.** Utilisation d'une biomolécule selon la revendication 7 dans un test de liaison par bioaffinité spécifique en utilisant une détermination flurométrique ou fluorométrique à résolution temporelle d'une luminescence spécifique.

**10.** Utilisation selon la revendication 9, dans laquelle le test de biaffinité spécifique est choisi parmi un test immunologique hétérogène, un test immunologique homogène, un test d'hybridation d'ADN, un test de liaison de récepteur, un test immunologique et un test immunohistochimique.

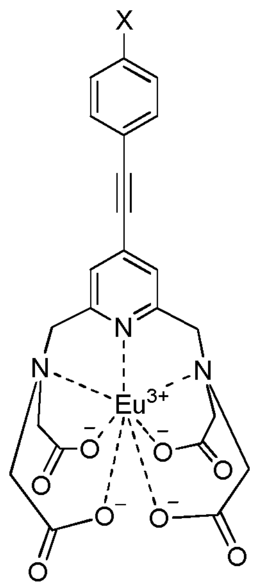

**1a,b,c**

**a**: X = NCS
**b**: X = $NHCSNHCH_2COOH$
**c**: X = $NHCSNH(CH_2)_3NH$-Biotin

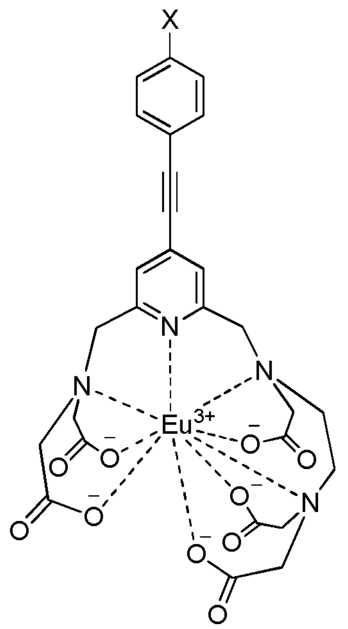

**6a,b,c**

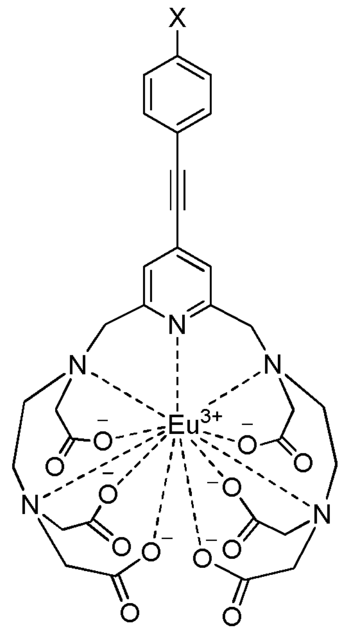

**7a,b,c**

**Fig. 1**

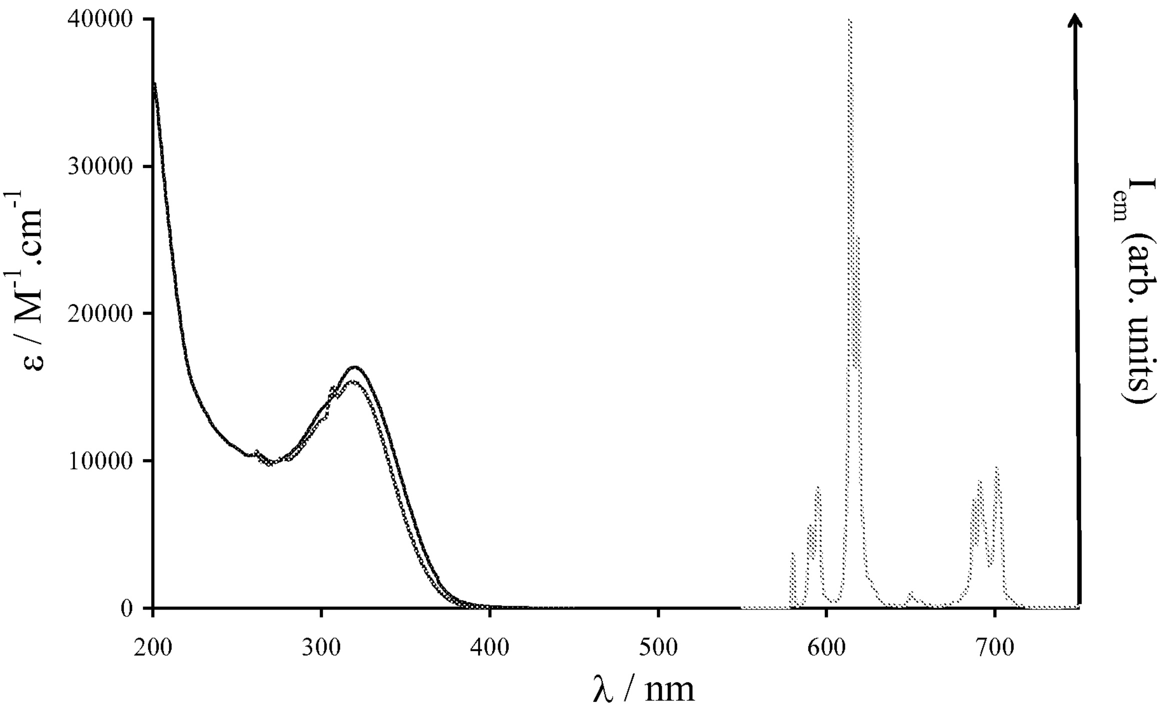
40000
30000
20000
10000
0
ε / M-1.cm-1
200
300
400
500
600
700
λ / nm
Iem (arb. units)

**Fig. 2**

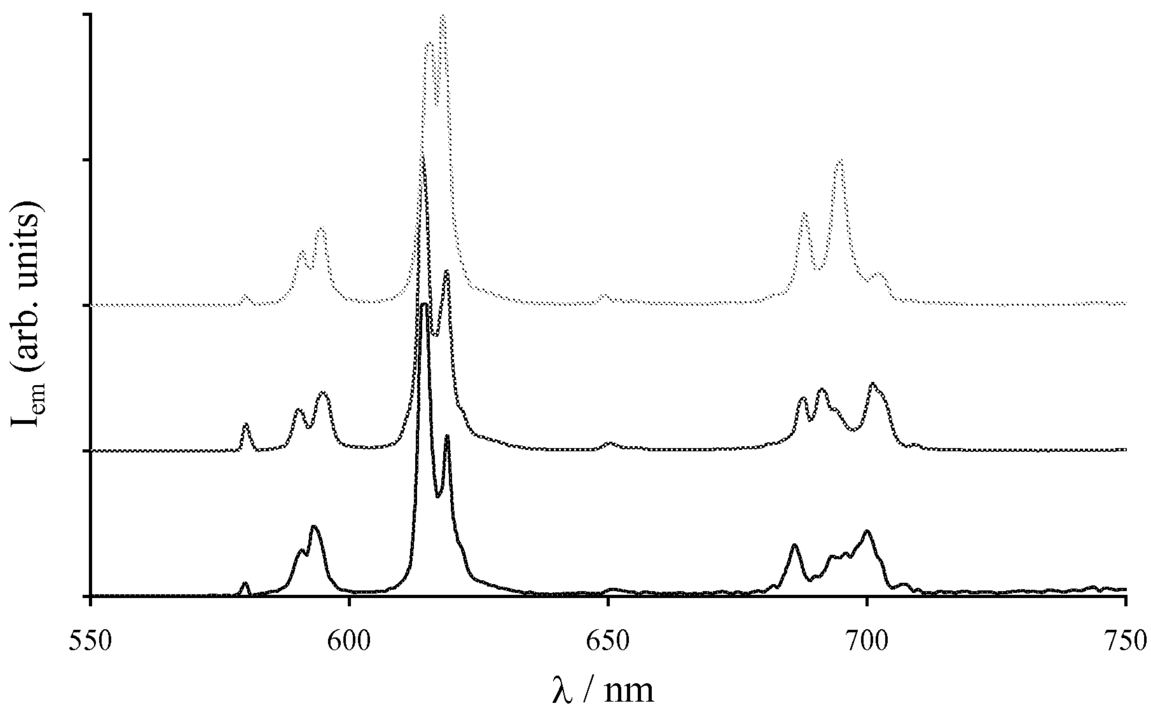
Iem (arb. units)
550
600
650
700
750
λ / nm

**Fig. 3**

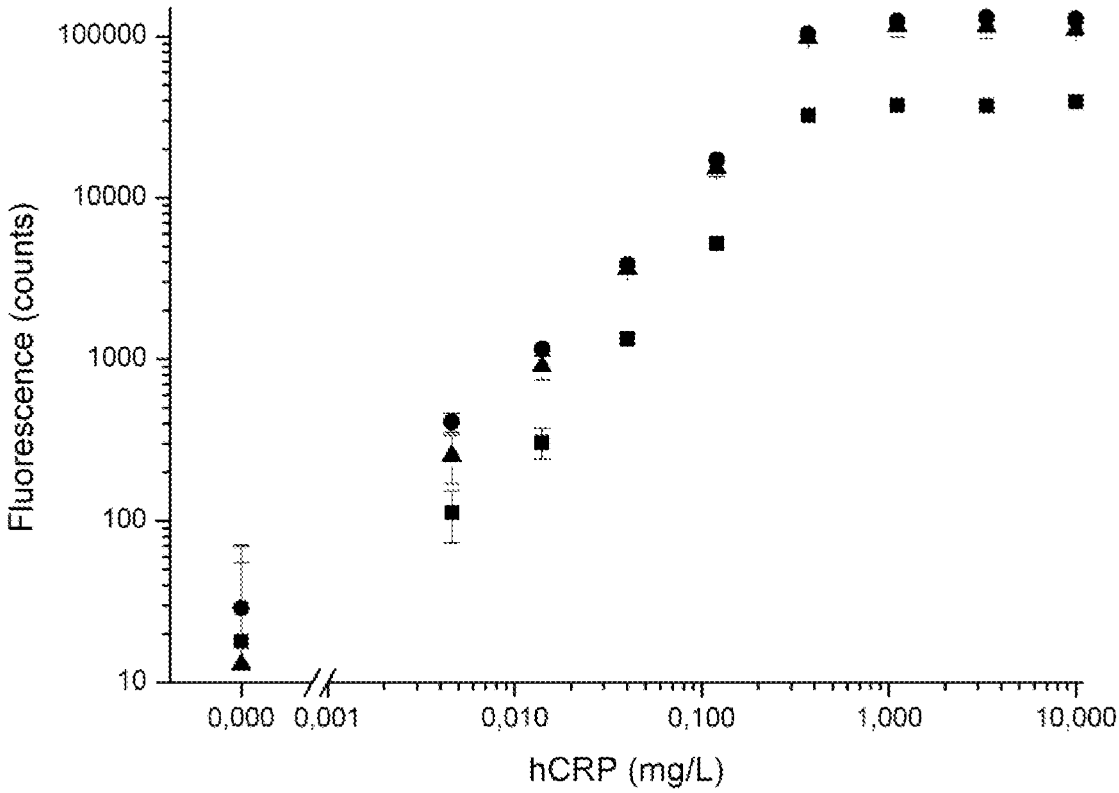
100000
10000
1000
100
10
Fluorescence (counts)
0,000
0,001
0,010
0,100
1,000
10,000
hCRP (mg/L)

**Fig. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4920195 A
- WO 2005058877 A
- WO 2005021538 A
- WO 2009030819 A
- WO 2008020113 A
- US 2004166585 A
- WO 2010055207 A
- US 5985566 A
- US 4761481 A
- US 004061 A


### Non-patent literature cited in the description


- *J. Luminescence,* 2005, vol. 113 (1-2), 17-26
- Bioconjugate Chemistry. 2009, vol. 20, 405
- **HEMMILÄ et al.** *J. Biochem. Biophys. Methods,* 1993, vol. 26, 283
- *Bioconjugate Chem.,* 2008, vol. 19, 1505-1509